# EUROPEAN PATENT SPECIFICATION

(11) **EP 3 295 922 B1**
(45) Date of publication and mention of the grant of the patent: **17.04.2024**
(21) Application number: 17199987.3
(22) Date of filing: 20.05.2014
(51) Int. Cl.: A61K 8/26, A61K 8/44, A61Q 15/00, A61K 8/19, C01F 7/56, A61K 8/06

(54) **ANTIPERSPIRANT COMPOSITIONS**
SCHWEISSHEMMENDE ZUSAMMENSETZUNGEN
COMPOSITIONS ANTI-TRANSPIRANTES

(30) Priority: 20.05.2013 EP 13168418; 20.05.2013 EP 13168417; 19.05.2014 EP 14168831
(43) Date of publication of application: 21.03.2018
(62) Divisional of application: 14725433.8
(73) Proprietor: Unilever IP Holdings B.V., 3013 AL Rotterdam (NL); Unilever Global IP Limited, Wirral, Merseyside CH62 4ZD (GB)
(72) Inventor: FAWZY, Karim Mohamed Anwar M, County Antrim, BT28 2UH (GB); FRANKLIN, Kevin Ronald, Wirral, Merseyside CH47 0LQ (GB); WATERFIELD, Peter Christopher, Bebington, Wirral, Merseyside CH63 3JW (GB)
(74) Representative: Whaley, Christopher

(56) References cited:
- EP-A2- 0 308 937
- EP-A2- 0 405 598
- EP-A2- 2 481 392
- WO-A2-00/10512
- WO-A2-2008/063188
- WO-A2-2012/010684
- WO-A2-2014/095688
- US-A- 4 359 456
- US-A- 4 435 382
- US-A1- 2004 115 147
- US-A1- 2007 020 211
- US-A1- 2007 196 303
- US-A1- 2007 286 830
- US-A1- 2009 317 347
- US-A1- 2011 038 823
- US-A1- 2011 038 902

## Description

The present invention is concerned with antiperspirant compositions and with methods of making the same. It is particularly concerned with compositions comprising basic aluminium chloride (herein BAC) antiperspirant actives and their manufacture.

The compositions of the present invention may be used as antiperspirant compositions and/or may be used in the manufacture of high efficacy antiperspirant compositions. Using the processes described herein, particularly effective or "activated" BAC compositions may be prepared.

Certain activated BAC actives are commercially available and their preparation and use are disclosed in numerous publications.

Traditionally, activated BAC samples have been prepared by prolonged heating of BAC solutions followed by spray drying; see, for example, US 4,359,456 (Gosling). The samples prepared by this method needed to be formulated into essentially anhydrous compositions in order for the antiperspirant to maintain its high activity.

Activated BAC samples have also been prepared using water soluble calcium acids, particularly with a further adjunct such as an amino acid, hydroxyl acid, or betaine. Some of these samples could be formulated into aqueous compositions without the antiperspirant losing all of its enhanced activity.

EP 1,104,282 (Gillette) discloses a means of producing activated BAC samples using a water soluble calcium salt and an amino acid or a hydroxy acid.

US 6,911,195 (Gillette) discloses water-in-oil emulsion gels comprising aluminium-zirconium antiperspirant salts activated using calcium ions.

US 5,955,065 (Gillette) discloses anhydrous suspension formulations comprising particulate BAC and aluminium-zirconium antiperspirant salts activated using calcium ions.

US 6,942,850 (Gillette) discloses aqueous alcoholic composition comprising aluminium-zirconium antiperspirant salts activated using calcium ions.

WO 2009/044381 (P&G) discloses water-in-oil emulsion sticks comprising BAC and aluminium-zirconium antiperspirant salts activated using calcium ions.

US 7,704,531 (Colgate) discloses compositions comprising an active system made from combining an aluminium or aluminium-zirconium salt, a calcium salt, and a betaine.

US 2011/0038823 (Dial/Henkel) discloses water-in-oil emulsion sticks comprising an antiperspirant active prepared by combining BAC, calcium chloride and glycine.

US 2007/196303, US 2007/0020211, WO 2008/063188, US 2008/0131354 and US 7,087,220 (Summit and Reheis) each describe methods of making calcium-activated antiperspirant salts.

WO 2009/075678, WO 2009/076592, WO 2011/016807, WO 2012/060817, WO 2012/061280, WO 2012/148480 and WO 2012/148481 (Colgate) disclose the manufacture of activated antiperspirant salts by neutralisation of aluminium chloride with calcium hydroxide in the presence of glycine.

The present invention is particularly concerned with BAC compositions comprising aluminium sesquichlorohydrate (herein ASCH) of chemical formula Al₂OH_{4.4}Cl_{1.6} to Al₂OH_{4.9}Cl_{1.1}. This material is commercially available, but its formulation and use described herein are new and deliver unexpected benefits.

In a first aspect of the present invention, there is provided the use of a basic aluminium chloride salt of formula Al₂OH_{4.4}Cl_{1.6} to Al₂OH_{4.9}Cl_{1.1} to deliver excellent antiperspirancy and reduced staining on clothes when delivered from an aqueous composition comprising said basic aluminium chloride salt, a water soluble calcium salt, and an amino acid, wherein the wherein the composition has a molar ratio of calcium to aluminium that is at least 1:20 and a molar ratio of amino acid to aluminium that is at least 1:10.

Herein, the "activation mixture" refers to the mixture of basic aluminium chloride salt of formula Al₂OH_{4.4}Cl_{1.6} to Al₂OH_{4.9}Cl_{1.1}, water soluble calcium salt, amino acid, and water.

The choice of BAC salt used is critical to the success of the present invention. We have found that surprisingly good results are found on using BAC salts commonly referred to as aluminium sesquichlorohydrate (herein ASCH) having the chemical formula Al₂OH_{4.4}Cl_{1.6} to Al₂OH_{4.9}Cl_{1.1}. Most commercial ASCH samples are of chemical formula Al₂OH_{4.7}Cl_{1.3} to Al₂OH_{4.9}Cl_{1.1} and it is preferred to use BAC salts of this formula.

The surprisingly good results referred to in the above paragraph include surprisingly good antiperspirancy performance. In addition, compositions prepared according to the present invention have remarkable storage stability, maintaining their good performance for many months.

A surprising benefit of compositions of the present invention is that they give minimal levels of clothes staining, particularly minimal yellow marks. The staining of clothes worn in proximity to areas of the body where currently marketed antiperspirant compositions have been applied is a well known problem. A partial solution to this problem is to reduce the amount of antiperspirant active applied; however, this tends to have the predictable effect of also reducing antiperspirant efficacy. The present inventors have found that by use of the compositions of the invention, yellows marks may be reduced/minimised without unduly compromising antiperspirancy. Indeed, the compositions of the present invention may deliver surprisingly reduced yellow marks with parity antiperspirancy compared to conventional compositions.

The reduced staining and in particular reduced yellowing benefit is of greatest relevance with regard to clothes worn in close proximity to areas where the antiperspirant composition has been applied, such as the underarm areas or axillae. The yellow staining is believed to result from an interaction between the sweat on the surface of the human body and the antiperspirant active applied. As such, the level of staining is particularly sensitive to the nature of the antiperspirant active applied. It remains unresolved as to why the antiperspirant active used in the compositions of the present invention gives the benefits that it does with regard to low/reduced staining/yellow marks.

The reduced (yellow) staining benefit occurs with clothes worn in close proximity to areas of the human body where the antiperspirant composition has been applied, but the benefit may be demonstrated by direct application of antiperspirant compositions onto fabrics, this method merely removing the intermediacy of the skin in the transfer of the antiperspirant from the composition to the fabric. In using this method, it is advantageous to also treat the fabric with human sweat or an artificial version thereof, to better show the combined effect of both sweat and antiperspirant on the fabric.

There are also sensory benefits, in particular a "reduced stickiness" benefit on use of the compositions of the present invention. The present inventors have found that by use of the compositions of the invention, the sticky feeling during and/or after application may be reduced/minimised without unduly compromising antiperspirancy. Indeed, the compositions of the present invention may deliver surprisingly reduced stickiness with parity antiperspirancy compared to conventional compositions.

The stickiness of skin during and/or following the application of aqueous roll-on antiperspirant compositions is a well known problem. It is related to the drying down of such compositions on the surface of the human body, particularly when the composition has been applied to the underarm areas or axillae. The antiperspirant active system used is one of many factors that can influence the stickiness perceived.

In a further aspect of the present invention, there is provided the use of a composition according to the first aspect of the invention to deliver excellent antiperspirancy and reduced stickiness.

The BAC salt used in the present invention has aluminium to chloride molar ratio of from 1.25:1 to 1.82:1 and preferably 1.54:1 to 1.82:1.

In order for the antiperspirant to become activated, it is important to have sufficient calcium present relative to the amount of aluminium present. The molar ratio of calcium to aluminium is at least 1:20. It is not advantageous to have the calcium concentration in excess of the aluminium concentration, indeed it is preferred that the calcium concentration is no more than half that of the aluminium concentration and more preferred that it is no more than a fifth of said concentration. It is independently preferred that this ratio is no greater than 1:2 and more preferred that it is no greater than 1:5.

In particularly preferred embodiments, the molar ratio of calcium to aluminium is at least 1:15 and preferably no greater than 1:5 and in especially preferred embodiments it is at least 1:10 and preferably no greater than 1:5.

A preferred water soluble calcium salt for use in the present invention is calcium chloride.

Herein, references to molar amounts and ratios of "aluminium" are calculated on the basis of mono-nuclear aluminium, but include aluminium present in poly-nuclear species; indeed, most of the aluminium in the salts of relevance is present in poly-nuclear species.

In order for the antiperspirant to become activated, it is important to have sufficient amino acid present relative to the amount of aluminium present. The molar ratio of amino acid to aluminium is at least 1:10 and most preferably at least 1:5. It is not advantageous to have the amino acid concentration in excess of the aluminium concentration; hence, the molar amino acid to aluminium is preferably from 1:10 to 1:1 and most preferably from 1:5 to 1:1.

In particularly preferred embodiments, the molar ratio of amino acid to aluminium is at least 1:4 and preferably no greater than 1:1 and in especially preferred embodiments it is at least 1:3 and preferably no greater than 1:1.

The presence of both calcium and amino acid is essential for the success of the present invention. In particularly preferred embodiments the molar ratio of calcium to aluminium is from 1:20 to 1:5 and the molar ratio of amino acid to aluminium is from 1:10 to 1:1.

In certain especially preferred embodiments, the molar ratio of calcium to aluminium is from 1:15 to 1:5 and the molar ratio of amino acid to aluminium is from 1:4 to 1:1. In these especially preferred embodiments, exemplary performance in is obtained when the molar ratio of calcium to aluminium is from 1:10 to 1:5 and the molar ratio of amino acid to aluminium is from 1:3 to 1:1.

The above indicated preferences for calcium to aluminium molar ratio and/or amino acid to aluminium molar ratio lead to compositions of higher Band III content (*vide infra*) and, in general, higher antiperspirancy performance. It will be noted that higher Band III content is generally indicative of higher antiperspirancy performance.

It is noteworthy that an amino acid must be used in order to activate the antiperspirant salt. The combination of a water soluble calcium salt and a hydroxy acid, as disclosed in EP 1,104,282 (Gillette), was found to be unsuccessful (*vide infra*)*.* Preferred amino acids for use in the present invention are glycine, alanine, valine and proline. A particularly preferred amino acid for use in the present invention is glycine.

The activation process generally produces a mixture of aluminium species having a relatively high content of what is commonly termed Band III material, as determined by SEC (Size Exclusion Chromatography) analysis. The SEC technique employed is well known in the art and is described in further detail in US 4,359,456 (Gosling). The SEC band commonly referred to as Band III is designated as "Peak 4" in EP 1,104,282 B1 by Gillette.

Herein, "Band III content" refers to the integrated area in the Band III region of the SEC chromatograph relative to the total integrated area in all of the regions corresponding to aluminium species; that is to say, Bands I, II, III, and IV.

In particular embodiments of the invention, compositions according to the invention intended for use as antiperspirant compositions preferably have a Band III content of at least 30%, more preferably at least 50% and most preferably at least 60%.

In the activation process and method of manufacture described herein, it is preferred that the activation mixture is heated for sufficient time for the Band III content of the aluminium species to become at least 30%, more preferably at least 40% and most preferably at least 50%.

In the activation process and method of manufacture described herein, the activation mixture is heated to at least 65°C, preferably to at least 75°C, and more preferably to at least 85°C.

In a preferred embodiment, the activation process and method of manufacture uses "ultra-high temperature" (UHT) processing, as commonly used in pasteurisation and sterilisation of milk. Such processing involves heating the activation mixture in excess of 100°C at a pressure of greater than 1 Bar (100,000 Pa). Using this method, we have found that the ASCH can be activated in a much shorter period of time; hence, the preferred heating time is less than one hour, more preferably less than 30 minutes, and most preferably less than 10 minutes.

In a preferred UHT process, the activation mixture is heated in excess of 110°C at pressure of greater than 2 Bar (200,000 Pa). This can enable activation of the ASCH in a matter of minutes or seconds, so the preferred heating time is less than one hour, more preferably less than 30 minutes, and most preferably less than 10 minutes.

Surprisingly, it was found that antiperspirant compositions prepared using the UHT process did not require a particularly high content of Band III material to give excellent antiperspirant performance (*vide infra*)*.* Indeed, antiperspirant compositions prepared by this method may have a Band III of 30% or greater.

In preferred methods of manufacture, particularly UHT processes, the concentration of aluminium used in the process is preferably from 0.5 to 5 mol.dm⁻³ and more preferably from 1.4 to 5 mol.dm⁻³. In certain particularly preferred embodiments, the concentration of aluminium may be over 3 mol.dm⁻³, particularly 3 to 4.5 mol.dm⁻³.

The processes described herein produce an aqueous solution of an activated antiperspirant salt. It will be realised, however, that such solutions may be dried by techniques known in the art, notably spray drying, to give a dried antiperspirant salt. Such dried antiperspirant salts may be used in a variety of compositions, including aerosols, sticks and soft solids. Such compositions are also to be considered antiperspirant compositions according to the invention. It will be realised that such compositions may be essentially anhydrous, having less than 1% by weight of free water or may be anhydrous, having less than 0.1% by weight of free water.

Herein, "free water" excludes any water of hydration associated with the antiperspirant salt or other component added to a particular composition, but includes all other water present.

Herein, compositions according to the invention intended for use as antiperspirant compositions are termed "antiperspirant compositions".

Other components may also be including in antiperspirant compositions according to the invention.

Herein, amounts and concentrations of ingredients are percentages by weight of the total composition, unless otherwise indicated and ratios are ratios by weight.

A preferred additional component of compositions of the invention is an oil.

Herein, the terms "oil" and signifies a water-insoluble organic material that is liquid at 20°C. Any material having a solubility of less than 0.1g/100g at 20°C is considered to be insoluble.

Herein "aqueous compositions" are compositions having a continuous phase that is predominately water; that is to say, greater than 50% water.

A preferred oil for use in accordance with the present invention is a fragrance oil, sometimes alternatively called a perfume oil. The fragrance oil may comprise a single fragrance or component more commonly a plurality of fragrance components. Herein, fragrance oils impart an odour, preferably a pleasant odour, to the composition. Preferably, the fragrance oil imparts a pleasant odour to the surface of the human body the composition is applied to the same.

The amount of fragrance oil in the composition is commonly up to 3% advantageously is at least 0.5% and particularly from 0.8% to 2%.

The total amount of oil in the composition is preferably from 0.1 to 20%, more preferably from 0.5 to 10%, and most preferably at from 2 to 8% by weight of the total composition. In certain preferred embodiments, particularly those also comprising an aluminium and/or zirconium containing antiperspirant active, the oil is present at greater than 2.5% and less than 6% by weight of the total composition.

In certain embodiments, it is preferred to include an oil, other than a fragrance oil, that has a relatively low viscosity, by which is meant less 250 cS (mm².s⁻¹). Such oils can improve the sensory properties of the composition on application and can lead to other benefits such as emolliency.

Suitable oils can be selected from alkyl ether oils having a boiling point of above 100°C and especially above 150°C, including polyalkyleneglycol alkyl ethers. Such ethers desirably comprise between 10 and 20 ethylene glycol or propylene glycol units and the alkyl group commonly contains from 4 to 20 carbon atoms. The preferred ether oils include polypropylene glycol alkyl ethers such as PPG-14-butylether and PPG-15-stearyl ether.

Suitable oils can include one or more triglyceride oils. The triglyceride oils commonly comprise the alkyl residues of aliphatic C₇ to C₂₀ alcohols, the total number of carbon atoms being selected in conjunction with the extent of olefinic unsaturation and/or branching to enable the triglyceride to be liquid at 20°C. One example is jojoba oil. Particularly preferably, in the triglyceride oil the alkyl residues are linear C₁₈ groups having one, two or three olefinic degrees of unsaturation, two or three being optionally conjugated, many of which are extractable from plants (or their synthetic analogues), including triglycerides of oleic acid, linoleic acid, conjugated linoleic acids, linolenic acid, petroselenic acid, ricinoleic acid, linolenelaidic acid, trans 7-octadecenoic acid, parinaric acid, pinolenic acid, punicic acid, petroselenic acid and stearidonic acid.

Suitable oils can include those derived from unsaturated C₁₈ acids, including coriander seed oil, impatiens balsimina seed oil, parinarium laurinarium kernel fat oil, sabastiana brasilinensis seed oil, dehydrated castor seed oil, borage seed oil, evening primrose oil, aquilegia vulgaris oil, sunflower (seed) oil and safflower oil. Other suitable oils are obtainable from hemp, and maize corn oil. An especially preferred oil by virtue of its characteristics is sunflower (seed) oil.

Further suitable oils, that can also be emollient oils, comprise alkyl or alkyl-aryl ester oils having a boiling point of above 150°C (and a melting point of below 20°C). Such ester oils include oils containing one or two alkyl groups of 12 to 24 carbon atoms length, including isopropyl myristate, isopropyl palmitate and myristyl palmitate. Other non-volatile ester oils include alkyl or aryl benzoates such C₁₂₋₁₅ alkyl benzoate, for example Finsolv TN^{™} or Finsolv Sun^{™}.

A further class of suitable oils comprises non-volatile dimethicones, often comprising phenyl or diphenylene substitution, for example Dow Corning 200 350cps or Dow Corning 556.

A preferred component in many antiperspirant compositions, particularly aqueous antiperspirant compositions, according to the invention is an emulsifier. Emulsifiers are particularly advantageous in aqueous systems additionally comprising fragrance oil and/or other oil.

Preferred compositions according to the invention are oil-in-water emulsions comprising an emulsifier, such compositions giving especially effective antiperspirancy, especially when the molar ratio of calcium to aluminium and/or amino acid to aluminium is within the preferred ranges indicated above (*vide supra*)*.*

It is preferred that emulsifiers used in aqueous antiperspirant compositions of the present invention form a lamellar phase emulsifier system in the composition. Such systems may be readily identified by means of optical microscopy. Such systems lead to good emulsion stability in compositions according to the invention.

It is preferred that aqueous antiperspirant compositions of the present invention comprise a non-ionic emulsifier system. Such an emulsifier system conveniently has a mean HLB value in the region of from about 5 to about 12 and particularly from 6 to about 10. In the preferred embodiments referred to in the paragraph immediately above, an especially desired mean HLB value is from 6 to 9. Such a mean HLB value can be provided by selecting an emulsifier having such an HLB value, or more preferably by employing a combination of at least two emulsifiers, a first (lower) HLB emulsifier having an HLB value in the range of from 2 to 6.5, such as in particular from 4 to 6 and a second (higher) HLB emulsifier having an HLB value in the range of from about 6.5 to 18 and especially from about 12 to about 18. When a combination of emulsifiers is employed, the average HLB value can be calculated as a weight average of the HLB values of the constituent emulsifiers.

Lamellar phase emulsifier systems preferably comprise two non-ionic surfactants, optionally selected as suggested in the paragraph immediately above. In a particular embodiment a first emulsifier is a fatty alcohol, such as cetyl and/or stearyl alcohol and a second emulsifier is much more hydrophilic, having a HLB of from about 6.5 to 18 and especially from about 12 to about 18.

An especially desirable range of emulsifiers comprises a hydrophilic moiety provided by a polyalkylene oxide (polyglycol), and a hydrophobic moiety provided by an aliphatic hydrocarbon, preferably containing at least 10 carbons and commonly linear. The hydrophobic and hydrophilic moieties can be linked via an ester or ether linkage, possibly via an intermediate polyol such as glycerol. A preferred range of emulsifiers comprises polyethylene glycol ethers.

Preferably the hydrophobic aliphatic substituent contains at least 12 carbons, and is derivable from lauryl, palmityl, cetyl, stearyl, and behenyl alcohol, and especially cetyl, stearyl or a mixture of cetyl and stearyl alcohols or from the corresponding carboxylic acids.

The polyalkylene oxide is often selected from polyethylene oxide and polypropylene oxide or a copolymer of ethylene oxide and especially comprises a polyethylene oxide. The number of alkylene oxide and especially of ethoxylate units within suitable emulsifiers is often selected within the range of from 2 to 100. Emulsifiers with a mean number of ethoxylate units in the region of 2 can provide a lower HLB value of below 6.5 and those having at least 4 such units provide a higher HLB value of above 6.5 and especially those containing at least 10 ethoxylate units which provide an HLB value of above 10. A preferred combination comprises a mixture of an ethoxylate containing 2 units and one containing from 10 to 40 units, such as from 15 to 30 or desirably from 20 to 25. Particularly conveniently, the combination of emulsifiers comprises steareth-2 and a selection from steareth-15 to steareth-30.

It is desirable to employ a mixture of ethoxylated alcohol emulsifiers in a weight ratio of emulsifier having a lower HLB value of less than 6.5 to emulsifier having a higher HLB value of greater than 8 of from 2:1 to 6:1 and particularly from 4:1 to 6:1.

The total proportion of emulsifiers in the composition is usually at least 1% and particularly at least 2% by weight. Commonly, the emulsifiers are not present at above 10%, often not more than 7% by weight and in many preferred embodiments up to 6% by weight. An especially desirable concentration range for the emulsifiers is from 2.5 to 5% by weight.

Other components that may be present include short chain (C₂-C₄) alcohols and especially polyols such glycerol, ethylene glycol, propylene glycol and polymers thereof, in particular polyethylene glycol) and polypropylene glycol). Poly(ethylene glycol) of average molecular weight 200 to 600 is a preferred component. Such components may add to the sensory properties of the composition and, when included, are typically present at from 0.5 to 10% of the total composition.

The aqueous compositions used in the present invention are very suitable for dispensing via a roll-on dispenser, for example any upright dispenser such as described in EP1175165 or an invert dispenser such as described in US6511243 or WO05/007377. Invert indicates that the dispenser stands stably with its dispensing ball below the formulation reservoir. In using such dispensers, the composition is applied by rolling the ball of the dispenser across the skin surface, depositing a film of fluid on the skin. Commonly the dispenser is wiped across the skin between 4 and 10 strokes. Commonly from 0.2 to 0.5g of the composition is deposited in each armpit per application.

The method of attaining an antiperspirant benefit described herein (*vide supra*) may involve direct or indirect topical application to the composition surface of the human body. In a related method, a composition comprising an antiperspirant salt prepared by drying an antiperspirant solution prepared according to the second aspect of the invention may be topically applied to the surface of the human body, directly or indirectly. In each of the methods described in this paragraph, the composition is preferably applied to the underarm regions of the human body.

### Examples

In the following examples, all percentages are by weight, unless otherwise indicated.

The Chlorohydrol 50 solution was an aqueous solution comprising approximately 50% by weight of aluminium chlorohydrate (ACH) and was obtained from SummitReheis. We measured its Al content at 12.9% by weight. The ACH has an approximate general formula Al₂(OH)₅Cl and an Al:Cl ratio of approximately 2:1.

The Reach 301 powder was approximately 100% ASCH and was obtained from SummitReheis. We measured its Al content at 24.1% by weight. The ASCH had an approximate general formula of Al₂(OH)_{4.8}Cl_{1.2} and an Al:Cl ratio of approximately 1.67:1.

The Aloxicoll 31L solution was an aqueous solution comprising approximately 50% by weight of ASCH and was obtained from BK Giulini GmbH. We measured its Al content at 11.9% by weight. The ASCH had an approximate general formula of Al₂(OH)_{4.8}Cl_{1.2} and an Al-Cl ratio of approximately 1.67:1.

The anhydrous calcium chloride and glycine were ex Sigma-Aldrich.

A range of antiperspirant (AP) salt solutions (1-12) was prepared as follows.

### Solution 1

30 parts of Chlorohydrol 50 solution was combined with 63.8 parts water at room temperature. The resulting solution had a Band III content of 15%.

### Solution 2

15 parts of Reach 301 powder was dissolved in 78.9 parts water at room temperature. The resulting solution had a Band III content of 27%.

### Solution 3

30 parts of Chlorohydrol 50 solution, 1.5 parts anhydrous calcium chloride and 4.7 parts glycine were combined with 57.6 parts water at room temperature. The resulting solution was heated at 85°C for 18hrs in a capped glass vessel and was then allowed to cool to ambient temperature. The resulting solution had a Band III content of 55%.

### Solution 4

15 parts of Reach 301 powder, 1.5 parts anhydrous calcium chloride and 4.7 parts glycine were combined with 72.6 parts water at room temperature. The resulting solution was heated at 85°C for 18hrs in a capped glass vessel and was then allowed to cool to ambient temperature. The resulting solution had a Band III content of 62%.

### Solution 5

7.5 parts of Reach 301 powder, 0.75 parts anhydrous calcium chloride and 2.35 parts glycine were combined with 83.2 parts water at room temperature. The resulting solution was heated at 85°C for 18hrs in a capped glass vessel and was then allowed to cool to ambient temperature. The resulting solution had a Band III content of 63%.

### Solution 6

15 parts of Reach 301 powder, 0.9 parts anhydrous calcium chloride and 2.0 parts glycine were combined with 75.9 parts water at room temperature. The resulting solution was heated at 85°C for 18hrs in a capped glass vessel and was then allowed to cool to ambient temperature. The resulting solution had a Band III content of 42%.

### Solution 7

5 parts of Reach 301 powder, 0.3 parts anhydrous calcium chloride, and 0.67 parts glycine were combined with 87.83 parts water at room temperature. The resulting solution was heated at 85°C for 18hrs in a capped glass vessel and was then allowed to cool to ambient temperature.

### Solution 8

15 parts of Reach 301 powder, 1.5 parts anhydrous calcium chloride and 4.7 parts glycine were combined with 72.6 parts water at room temperature. The resulting solution was heated to 115°C for 50 seconds and maintained at this temperature for a further 194 seconds in UHT processing equipment at a pressure of 3 to 7 Bar, and was then cooled over 56 seconds. The resulting solution had a Band III content of 52%.

### Solution 9

30 parts of Reach 301 powder, 3.0 parts anhydrous calcium chloride and 9.4 parts glycine were combined with 57.6 parts water at room temperature. The resulting solution was heated at 85°C for 18hrs in a capped glass vessel. The resulting solution had a Band III content of 69%.

### Solution 10

30 parts Aloxicoll 31L, 0.9 parts anhydrous calcium chloride and 2.0 parts glycine were combined with 2.6 parts water at room temperature. The resulting solution was heated to 135°C for 99 seconds and maintained at this temperature for 389 seconds in UHT processing equipment at a pressure of 3 to 7 Bar, and was then cooled, over 200 seconds. The resulting solution had a Band III content of 31%.

### Solution 11

60 parts of Chlorohydrol 50 solution was combined with 40 parts water at room temperature.

### Solution 12

30 parts of Reach 301 powder, 3.0 parts calcium chloride anhydrous (Sigma-Aldrich), 5.4 parts glycine (Sigma-Aldrich) were combined with 57.6 parts water at room temperature. The resulting solution was heated at 85°C for 18hrs in a capped glass vessel.

Each of solutions 1 to 8 was used in the preparation of an antiperspirant roll-on composition as indicated in Table 1. Each of solutions 9 and 10 was used in the preparation of antiperspirant roll-on compositions as indicated in Table 2. Each of solutions 11 and 12 was used in the preparation of an antiperspirant cream composition as indicated in Table 4.

The roll-on compositions of Table 1 were prepared at a 1.5 kg scale. The AP salt solution was placed in a large glass vessel. This was heated to 52°C while stirring with a Silverson mixer (2 inch head, square mesh, high shear screen) at 1500 rpm. The Steareth 20 was then added to the AP salt solution and allowed to dissolve. In a separate vessel the sunflower seed oil and Steareth 2 were combined and heated to 65°C with gently stirring. The stirring speed of the Silverson mixer on the main vessel was increased to 2500rpm and the mixture of sunflower seed oil and Steareth 2 added over 9 minutes while maintaining the temperature at 52°C. The temperature was then reduced to 42°C and the fragrance added. The Silverson speed was increased to 7500rpm for 3 minutes and then the resulting emulsion mixture was dispensed into standard roll-on packs.

**Table 1: Antiperspirant Roll-On Composition**

| Component: | % w/w |
|---|---|
| AP salt Solution 1 to 8 | 93.8 |
| Steareth 20 (1) | 0.9 |
| Steareth 2 (2) | 2.3 |
| Sunflower seed oil (3) | 2.0 |
| Fragrance | 1.0 |

| | |
|---|---|
| 1. Volpol S20, ex Croda. 2. Volpol S2A, ex Croda. 3. Akosun, ex AAK Karlshmans. | |

The roll-on compositions of Table 2 were prepared in exactly the same way as those in Table 1, except that the AP salt solution was pre-diluted with additional water as indicated.

**Table 2**

| Component: | % w/w | |
|---|---|---|
| Example: | 6 | 7 |
| AP salt Solution 9 | 50 | -- |
| AP salt Solution 10 | -- | 35.5 |
| Water | 43.8 | 58.3 |
| Steareth 20 (1) | 0.9 | 0.9 |
| Steareth 2 (2) | 2.3 | 2.3 |
| Sunflower Seed Oil (3) | 2.0 | 2.0 |
| Fragrance | 1.0 | 1.0 |

The roll-on compositions as described in the above two tables are summarised in Table 3.

"Sweat weight reduction" SWR results were obtained on use of each of the compositions using a test panel of 30 female volunteers. Test operators applied Comparative Example A (0.30g) to one axilla and 0.30g of non-antiperspirant deodorant body spray to the other axilla of each panellist. This was done once each day for three days. After the third application, panellists were requested not to wash under their arms for the following 24 hours.

24 hours after the third and final product application, the panellists were induced to sweat in a hot-room at 40°C (±2°C) and 40% (±5%) relative humidity, for 40 minutes. After this period, the panellists left the hot-room and their axillae were carefully wiped dry. Pre-weighed cotton pads were then applied to each axilla of each panellist and the panellists re-entered the hot-room for a further 20 minutes. Following this period, the pads were removed and re-weighed, enabling the weight of sweat generated to be calculated.

The sweat weight reduction (SWR) for each panellist was calculated as a percentage (% SWR) and the mean % SWR was calculated according to the method described by Murphy and Levine in "Analysis of Antiperspirant Efficacy Results", J. Soc. Cosmetic Chemists, 1991 (May), 42, 167-197.

**Table 3**

| Example | AP salt solution | AP salt in composition (Approximate %) | Molar ratio | Molar ratio |
|---|---|---|---|---|
| | | | Ca : Al | Glycine : Al |
| A | 1 | 15 | 0 | 0 |
| B | 2 | 15 | 0 | 0 |
| C | 3 | 15 | 1 : 10.6 | 1 : 2.3 |
| 1 | 4 | 15 | 1 : 9.9 | 1 : 2.1 |
| 2 | 5 | 7.5 | 1 : 9.9 | 1 : 2.1 |
| 3 | 6 | 15 | 1 : 16.5 | 1 : 5.0 |
| 4 | 7 | 5 | 1 : 16.5 | 1 : 5.0 |
| 5 | 8 | 15 | 1 : 9.9 | 1 : 2.1 |
| 6 | 9 | 15 | 1 : 9.9 | 1 : 2.1 |
| 7 | 10 | 15 | 1 : 16.3 | 1 : 5.0 |

For Comparative Examples B and C and Examples 1 to 7, the above procedure was repeated except that the product with which the test sample was compared was Comparative Example A.

Comparative example A, a BAC composition lacking calcium chloride and glycine, gave a SWR of 41%. Comparative example B, an ASCH composition also lacking calcium chloride and glycine, also gave a SWR of 41%. Comparative example B, a BAC composition having calcium chloride and glycine, gave a somewhat better SWR of 48%.

Example 1, an ASCH composition having calcium chloride and glycine, gave a significantly greater SWR than Comparative example A. Surprisingly, Example 2, having only half the AP salt level of Example 1, also gave a significantly greater SWR than Comparative Example A.

Example 1 was tested again after the sample had been stored for 12 weeks at 45°C. The SWR on this latter occasion was not significantly different to the earlier result, illustrating the excellent stability of examples according to the invention.

Examples 3 and 4 were prepared using much lower ratios of calcium to aluminium and glycine to aluminium compared with Examples 1 and 2. Nevertheless, Example 3 gave an excellent SWR and Example 4 was able to match the performance of Comparative examples A and B despite only having a third the level of AP salt present.

The cream compositions indicated in Table 4 were prepared using Solutions 11 and 12 (*vide supra*) at 1.5 kg scale. The water and glycerol were combined and heated to 80°C in a main vessel with gentle stirring (low speed scraper blade mixer). In a second vessel the glyceryl stearate, liquid petrolatum, cetearyl alcohol and Polawax GP200 were combined and heated 80°C with gentle stirring (magnetic stirrer bar). While maintaining the temperature at 80°C, the contents of the second vessel were then added slowly to the main vessel. The speed of the scraper blade mixer was adjusted throughout the addition to ensure good mixing at all times. While maintaining temperature and mixing, the titanium dioxide was then added and dispersed. The temperature was reduced to 50°C and the AP salt solution added. The temperature was reduced to 40°C and the fragrance added. Once fully mixed the formulation was placed in a suitable dispenser.

**Table 4**

| Component: | % w/w |
|---|---|
| AP salt solution 11 to 12 | 50 |
| Water | 32.6 |
| Glyceryl Stearate | 7.5 |
| Liquid Petrolatum (4) | 1.0 |
| Glycerol (5) | 1.5 |
| Titanium Dioxide (6) | 0.2 |
| Cetearyl alcohol (7) | 1.0 |
| Cetearyl alcohol, PEG-20 Stearate (8) | 5.0 |
| Fragrance | 1.2 |

| | |
|---|---|
| 4. Liquid petrolatum (Blanol, ex Evonik Degussa). 5. Glycerol (Pricerine 9091, ex Croda). 6. Titanium dioxide (Tiona AG, ex Aston Chemicals). 7. Cetearyl alcohol (Laurex CS, ex Huntsman). 8. Polawax GP200, ex Croda. | |

The cream compositions as described in the above table are summarised in Table 5.

**Table 5**

| Example | AP salt solution | AP salt in composition (Approximate %) | Molar ratio | Molar ratio |
|---|---|---|---|---|
| | | | | Glycine : Al |
| | | | Ca : Al | |
| D | 11 | 15 | 0 | 0 |
| 8 | 12 | 15 | 1 : 12.4 | 1 : 4.7 |

SWR results were obtained using the same method as described immediately below Table 3, Comparative Example D and Example 8 each being compared with a non-antiperspirant deodorant body spray. Example 8 gave a significantly better SWR figure than Comparative Example D.

In a further series of experiments, antiperspirant salt solutions analogous to Solution 4 as described above were prepared. The preparatory procedure for Solution 4*R* was exactly the same as for Solution 4. For Solution 4*LA*, the procedure was exactly the same except that the 4.7 parts glycine was replaced by 4.7 parts lactic acid. For Solution 4*GA* the procedure was exactly the same except that the 4.7 parts glycine was replaced by 4.7 parts glycolic acid. Following preparation, the solutions were analysed using SEC by a method based upon that described in US 4,359,456 by Gosling. The results are indicated below.

**Table 6**

| Solution | Band III content |
|---|---|
| 4*R* | 70% |
| 4*LA* | 27% |
| 4*GA* | 23% |

The results indicate that the amino acid glycine is more effective at activating the ASCH than either of the hydroxy acids.

## Claims

1. The use of a basic aluminium chloride salt of formula Al₂OH_{4.4}Cl_{1.6} to Al₂OH_{4.9}C_{1.1} to deliver excellent antiperspirancy and reduced staining on clothes when delivered from an aqueous composition comprising said basic aluminium chloride salt, a water soluble calcium salt, and an amino acid, wherein the composition has a molar ratio of calcium to aluminium that is at least 1:20 and a molar ratio of amino acid to aluminium that is at least 1:10.

2. The use according to claim 1 to deliver excellent antiperspirancy and reduced yellowing on clothes.

3. The use according to claim 1 or claim 2, wherein the composition has a molar ratio of calcium to aluminium that is at least 1:15 and a molar ratio of amino acid to aluminium that is at least 1:4.

4. The use according to any of the preceding claims, wherein the water soluble calcium salt is calcium chloride and the amino acid is glycine.

5. The use according to any of the preceding claims, wherein the composition is an oil-in-water emulsion comprising an emulsifier.

6. The use according to any of the preceding claims wherein the basic aluminium chloride salt is of formula Al₂OH_{4.7}Cl_{1.3} to Al₂OH_{4.9}Cl_{1.1}.

7. The use according to any of the preceding claims wherein the basic aluminium chloride has a Band III content measured by SEC of greater than 30%.

## Patentansprüche

1. Verwendung eines basischen Aluminiumchloridsalzes der Formel Al₂OH_{4,4}Cl_{1,6} bis Al₂OH_{4,9}Cl_{1,1}, um hervorragende schweißhemmende Wirkung und verminderte Fleckenbildung auf der Kleidung zu liefern, wenn es aus einer wässrigen Zusammensetzung zugeführt wird, umfassend das basische Aluminiumchloridsalz, ein wasserlösliches Calciumsalz und eine Aminosäure, wobei die Zusammensetzung ein Molverhältnis von Calcium zu Aluminium von mindestens 1:20 und ein Molverhältnis von Aminosäure zu Aluminium von mindestens 1:10 aufweist.

2. Verwendung nach Anspruch 1, um hervorragende schweißhemmende Wirkung und verringerte Vergilbung auf der Kleidung zu liefern.

3. Verwendung nach Anspruch 1 oder Anspruch 2, wobei die Zusammensetzung ein Molverhältnis von Calcium zu Aluminium von mindestens 1:15 und ein Molverhältnis von Aminosäure zu Aluminium von mindestens 1:4 aufweist.

4. Verwendung nach einem der vorhergehenden Ansprüche, wobei das wasserlösliche Calciumsalz Calciumchlorid ist und die Aminosäure Glycin ist.

5. Verwendung nach einem der vorhergehenden Ansprüche, wobei die Zusammensetzung eine Öl-in-Wasser-Emulsion ist, umfassend einen Emulgator.

6. Verwendung nach einem der vorhergehenden Ansprüche, wobei das basische Aluminiumchloridsalz die Formel Al_{2O}H_{4,7}Cl_{1,3} bis Al₂OH_{4,9}Cl_{1,1} aufweist.

7. Verwendung nach einem der vorhergehenden Ansprüche, wobei das basische Aluminiumchlorid einen mittels SEC gemessen Band III-Gehalt von mehr als 30% aufweist.

## Revendications

1. Utilisation d'un sel de chlorure d'aluminium basique de formule Al₂OH_{4,4}Cl_{1,6} à Al₂OH_{4,9}Cl_{1,1} pour délivrer une excellente caractéristique antiperspirante et une formation de tache réduite sur les vêtements lorsque délivrée à partir d'une composition aqueuse comprenant ledit sel de chlorure d'aluminium basique, un sel de calcium soluble dans l'eau, et un acide aminé, dans laquelle la composition présente un rapport molaire de calcium à aluminium qui est d'au moins 1:20 et un rapport molaire d'acide aminé à aluminium qui est d'au moins 1:10.

2. Utilisation selon la revendication 1 pour délivrer une excellente caractéristique antiperspirante et un jaunissement réduit aux vêtements.

3. Utilisation selon la revendication 1 ou revendication 2, dans laquelle la composition présente un rapport molaire de calcium à aluminium qui est d'au moins 1:15 et un rapport molaire d'acide aminé à aluminium qui est d'au moins 1:4.

4. Utilisation selon l'une quelconque des revendications précédentes, dans laquelle le sel de calcium soluble dans l'eau est le chlorure de calcium et l'acide aminé est la glycine.

5. Utilisation selon l'une quelconque des revendications précédentes, dans laquelle la composition est une émulsion huile-dans-eau comprenant un émulsifiant.

6. Utilisation selon l'une quelconque des revendications précédentes, dans laquelle le sel de chlorure d'aluminium basique est de la formule Al₂OH_{4,7}Cl_{1,3} à Al₂OH_{4,9}Cl_{1,1}.

7. Utilisation selon l'une quelconque des revendications précédentes, dans laquelle le chlorure d'aluminium basique présente une teneur de bande III mesurée par SEC supérieure à 30 %.
